(19) Europäisches Patentamt

European Patent Office

Office européen des brevets

(11) **EP 0 650 719 B1**

(12) **EUROPÄISCHE PATENTSCHRIFT**

(45) Veröffentlichungstag und Bekanntmachung des Hinweises auf die Patenterteilung:
**03.11.1999 Patentblatt 1999/44**

(51) Int. Cl.$^6$: **A61K 7/13**, A61K 7/135

(21) Anmeldenummer: **94113109.6**

(22) Anmeldetag: **23.08.1994**

(54) **Verfahren zur Herstellung eines persulfathaltigen Granulates zum Entfärben oder Blondieren von Haaren**

Process for the manufacture of a granulate containing persulphates for removing the color or bleaching of hair

Procédé de fabrication d'un granulé contenant des persulfates pour décolorer ou blanchir les cheveux

(84) Benannte Vertragsstaaten:
**DE ES FR GB IT**

(30) Priorität: **30.10.1993 DE 4337178**

(43) Veröffentlichungstag der Anmeldung:
**03.05.1995 Patentblatt 1995/18**

(73) Patentinhaber:
**Wella Aktiengesellschaft
64274 Darmstadt (DE)**

(72) Erfinder:
- **Clausen, Thomas, Dr.
  D-64665 Alsbach (DE)**
- **Balzer, Wolfgang R., Dr.
  D-64665 Alsbach (DE)**
- **Port, Volker, Dr.
  D-64807 Dieburg (DE)**
- **Kujawa, Jolanthe
  D-64289 Darmstadt-Kranichstein (DE)**

(56) Entgegenhaltungen:
WO-A-92/03120     DE-A- 2 023 922
US-A- 3 726 967

**Beschreibung**

Gegenstand der vorliegenden Erfindung ist ein Verfahren zur Herstellung eines granulatförmigen persulfathaltigen Mittels zum Entfärben oder Blondieren von Haaren sowie das durch dieses Verfahren hergestellte Mittel.

Zum Entfärben oder Blondieren von Haaren werden üblicherweise oxidierende Zubereitungen verwendet, welche durch Auflösen eines sogenannten Blondierpulvers (Pulvergemisch aus Alkalisalzen und anorganischen Persalzen, wie zum Beispiel Natrium- oder Ammoniumpersulfat) in einer wäßrigen Wasserstoffperoxidlösung erhalten werden.

Die Verwendung derartiger Blondierpulver, welche Zwangsläufig aus mehreren Bestandteilen bestehen, bringt jedoch eine Vielzahl von Nachteilen mit sich. So kommt es durch die Verwendung von Rohstoffen mit unterschiedlicher Dichte häufig während des Transportes oder der Lagerung zur Trennung der unterschiedlichen Pulverbestandteile, wobei sich die schweren Pulverbestandteile im unteren Teil und die leichteren Pulverbestandteile im oberen Teil des Behältnisses ansammeln. Diese Entmischung hat zur Folge, daß die gleiche Pulvermenge je nach ihrem Entnahmeort eine unterschiedliche chemische Zusammensetzung und somit auch eine unterschiedliche Blondierwirkung aufweisen kann.

Um dieser Entmischung entgegenzuwirken, ist es erforderlich, vor jeder Pulverentnahme das Pulver gründlich zu schütteln, was der Verwender in der Regel jedoch nicht tut.

Eine Entmischung kann auch durch den Einsatz von Pulvergemischen mit sehr kleiner Korngröße verhindert werden. Dies hat jedoch den Nachteil, daß solche Pulvergemische - insbesondere beim Öffnen der Behältnisse, bei der Entnahme des Pulvers oder beim Vermischen mit der Wasserstoffperoxidlösung - zu einer starken Staubentwicklung neigen, was zu Reizungen der Atmungsorgane führen kann. Weiterhin besitzten derartige Pulvergemische aufgrund der geringen Korngröße eine große Oberfläche, wodurch eine Aufnahme von Feuchtigkeit beim Öffnen und Schließen des Behältnisses und damit eine Verringerung der Blondierwirkung infolge der Desaktivierung des Sauerstoffträgers begünstigt wird.

Es wurden bereits zahlreiche Versuche unternommen, dieses Problem zu lösen.

So wird in der DE-OS 40 26 235 vorgeschlagen, eine Mischung bestehend aus einem Granulat der anorganischen Persulfate und einem Granulat der übrigen Bestandteile des Blondiermittels anstelle eines Blondierpulvers zu verwenden. Hierdurch kann zwar das Problem der Staubentwicklung behoben werden, das Problem der Entmischung kann auf diesem Wege jedoch nicht gelöst werden, da es technisch äußerst schwierig ist, Einzelgranulate mit identischer und konstanter Korngröße oder Schüttegewicht herzustellen. Weiterhin kann es aufgrund der unterschiedlichen Löslichkeit der einzelnen Granulate zu einer Beeinträchtigung der Blondierwirkung kommen. Es erscheint zudem auch aus ökonomischen Gesichtspunkten wenig sinnvoll, anstelle eines einzelnen Granulates eine Mischung aus mehreren Granulaten herzustellen.

In der DE-AS 20 23 922 wird empfohlen, ein Granulat anstelle des Pulvers zu verwenden. Dieses Granulat soll durch Besprühen des alle erforderlichen Bestandteile enthaltenden Blondierpulvers mit einer wäßrigen, alkoholischen oder wäßrig - alkoholischen Polymerlösung in einem geeigneten Mischer hergestellt werden.

Bei diesem Granulierverfahren kommt es jedoch zu einem starken Ammoniakverlust, wodurch die Blondierwirkung des Granulats verschlechtert wird. Dieser Ammoniakverlust soll durch die Erhöhung des Ammoniumsalzanteils in dem eingesetzten Pulver beziehungsweise durch den Zusatz von Ammoniak zu der Polymerlösung kompensiert werden. Da der verfahrensbedingte Ammoniakverlust bei diesem Granulationsverfahren stark schwankt, ist es mit diesem Verfahren nicht möglich, ein Granulat mit einer konstanten chemischen Zusammensetzung herzustellen.

Das vorgenannte Granulationsverfahren besitzt zudem zwei weitere Nachteile: Zum einen ist die Herstellung der benötigten homogenen, versprühbaren Bindemittellösung, welche eine bestimmte Viskosität aufweisen muß, sehr zeitaufwendig. Zum anderen ist für die Einbringung der erforderlichen Menge an Bindemittel eine relativ große Menge an Polymerlösung (Verhältnis Pulver: Polymerlösung = 1 : 1) erforderlich, was sowohl aus ökonomischen als auch ökologischen Gesichtspunkten - insbesondere bei der Verwendung von alkoholischen Lösungen - bedenklich erscheint, da ein hoher Energieaufwand für die Trocknung des Granulates beziehungsweise die Rückgewinnung des eingesetzten Alkohols erforderlich ist.

Zudem sind die nach diesem Verfahren hergestellten Granulate sehr hart und nur schwer in der Wasserstoffperoxidlösung dispergierbar.

Es bestand daher die Aufgabe, ein geeignetes granulatförmiges Mittel zum Entfärben oder Blondieren von Haaren sowie ein Verfahren zur Herstellung dieses Granulates zur Verfügung zu stellen.

Hierzu wurde nunmehr gefunden, daß die vorstehend beschriebenen Nachteile vermieden werden können, wenn bei der Granulation des Blondierpulvers das Bindemittel in das Blondierpulver eingearbeitet wird und dieses Gemisch anschließend mit einem geeigneten Lösungsmittel besprüht wird.

Gegenstand der vorliegenden Erfindung ist daher ein Verfahren zur Herstellung eines granulatförmigen Mittels zum Entfärben oder Blondieren von Haaren, welches dadurch gekennzeichnet ist, daß man in einer geeigneten Granuliervorrichtung bei Raumtemperatur über einem mindestens ein anorganisches Persulfat und übliche Hilfs- und Zusatzstoffe sowie ein Bindemittel enthaltenden pulverförmigen Gemisch einen Alkohol versprüht und anschließend das erhaltene Granulat trocknet.

Bei dem erfindungsgemäßen Verfahren wird zunächst das pulverförmigen Ausgangsgemisch durch Vermischen der anorganischen Persulfate mit den Hilfs- und Zusatzstoffen sowie dem Bindemittel hergestellt. Hierzu können handelsübliche Zwangsmischer, wie zum Beispiel nach dem Diosna-System arbeitende Mischer oder Pflugscharmischer, verwendet werden.

Sodann wird dieses Pulver unter Rühren in einer geeigneten Granulationsvorrichtung (beispielsweise den vorgenannten Zwangsmischern oder einer Granuliertrommel) mit dem Alkohol befeuchtet, wobei die Alkoholmenge so bemessen wird, daß ein angefeuchtetes,noch nicht zum Zusammenbacken neigendes Gemisch entsteht.

Das Gewichtsverhältnis des pulverförmigen Ausgangsgemisches zum Alkohol beträgt hierbei vorzugsweise 13 : 1 bis 4 : 1, wobei ein Gewichtsverhältnis von 10 : 1 bis 7 : 1 besonders bevorzugt ist.

Als Alkohol kann jeder beliebige leicht flüchtige Alkohol verwendet werden, dessen Siedepunkt nicht über etwa 85 Grad Celsius liegt und der ein ausreichendes Lösungsvermögen für das Bindemittel besitzt, wobei Ethanol, Isopropanol oder ein Gemisch dieser Alkohole bevorzugt ist.

Als Bindemittel können insbesondere Polyethylenglykole mit einem Molekulargewicht von 200 bis 10000 oder Polyvinylpyrrolidon verwendet werden.

Das Bindemittel wird in dem pulverförmigen Gemisch in einer Menge von 1 bis 20 Gewichtsprozent, vorzugsweise 3 bis 8 Gewichtsprozent, eingesetzt.

Neben dem Bindemittel enthält das pulverförmige Gemisch ein oder mehrere anorganische Persulfate, wie zum Beispiel Natriumpersulfat, Kaliumpersulfat oder Ammoniumpersulfat. Die Persulfate sind in dem Gemisch vorzugsweise in einer Menge von 30 bis 65 Gewichtsprozent enthalten, wobei eine Menge von 40 bis 60 Gewichtsprozent besonders bevorzugt ist.

Weiterhin kann das pulverförmige Gemisch alkalisch reagierende Alkali- oder Erdalkalisalze, wie zum Beispiel Natriumcarbonat, Natriumsilikat, Natriumhydrogencarbonat oder Magnesiumcarbonat, enthalten, wobei eine Einsatzmenge von 15 bis 45 Gewichtsprozent und insbesondere von 25 bis 40 Gewichtsprozent bevorzugt ist.

Darüber hinaus können in dem pulverförmigen Gemisch Chelatisierungsreagenzien für Schwermetallionen, beispielsweise Salze der Ethylendiaminotetraessigsäure, in einer Konzentration von 0,1 bis 3 Gewichtsprozent, vorzugsweise 0,5 bis 1,5 Gewichtsprozent, sowie Parfüme und Anfärbestoffe, beispielsweise aus den Klassen der Ultramarinfarbstoffe und sauren Farbstoffe, jeweils in einer Menge von bis zu 1 Gewichtsprozent enthalten sein.

Ebenfalls können in dem pulverförmigen Gemisch Netzmittel und Emulgatoren aus den Klassen der amphoteren, anionischen, kationischen oder nichtionischen oberflächenaktiven Substanzen, wie zum Beispiel Alkylbetaine, Fettalkoholsulfate, Fettalkoholethersulfate, Alkylsulfate, Alkylbenzolsulfonate, Alkyltrimethylammoniumsalze, oxethylierte Fettalkohole, oxethylierte Nonylphenole, oxethylierte Fettsäureester und Fettsäurealkanolamide, sowie wasserlösliche Verdicker, beispielsweise Stärken oder Celluloseether, wie zum Beispiel Methylcellulose, Ethylcellulose, Hydroxyethylcellulose, Methylhydroxyethylcellulose, Methylhydroxypropylcellulose und Carboxymethylcellulose, enthalten sein. Die Netzmittel und Emulgatoren können in einer Menge von 0,1 bis 15 Gewichtsprozent, vorzugsweise 0,1 bis 8 Gewichtsprozent eingesetzt werden, während die Einsatzmenge der wasserlöslichen Verdicker 0,1 bis 15 Gewichtsprozent, vorzugsweise 1 bis 10 Gewichtsprozent beträgt.

Das vorstehend beschriebene pulverförmige Gemisch wird sodann in einer geeigneten Granuliervorrichtung so lange behandelt, bis ein Granulat entstanden ist. Die Granulationszeit hängt hierbei im wesentlichen von dem verwendeten Granulatiosverfahren ab. In der Regel erfordert die Granulation etwa 10 bis 60 Minuten, wobei für die Mischgranulation etwa 10 bis 20 Minuten erforderlich sind, während bei der Wirbelschichtgranulation bedingt durch längere Sprühzeiten die Granulationszeit etwa 20 bis 60 Minuten beträgt.

Nach Beendigung des Granulationsvorgangs wird das Granulat in einem geeigneten Trockner, beispielsweise einem Hordentrockner, Trommeltrockner, Wirbelschichttrockner oder Vakuumtrockner, unter Erwärmen oder im Vakuum getrocknet, wobei eine Erwärmung des Granulates auf Temperaturen über 40 Grad Celsius zweckmäßigerweise vermieden werden sollte.

Nach Beendigung des Trockenvorganges sollte der Alkoholgehalt des Granulates weniger als 1 Gewichtsprozent betragen.

Der Durchmesser des erhaltenen Granulates liegt vorzugsweise in einem Bereich von 0,1 bis 2 mm, wobei ein Durchmesser von 0,2 bis 0,5 mm besonders bevorzugt ist.

Falls erforderlich können unerwünschte Fein- bzw. Grobkornanteile durch Aussieben mit kalibrierten Sieben abgetrennt werden, so daß ein für die Anwendung optimales Kornspektrum des Granulates verbleibt.

Die abgetrennten Fein- und Grobkornanteile können dem Herstellungsprozeß wieder zugeführt werden.

Das nach dem erfindungsgemäßen Verfahren hergestellte Granulat besitzt gegenüber den aus dem Stand der Technik bekannten Granulaten eine leichtere Dosierbarkeit, eine bessere Homogenität und eine bessere Dispergierbarkeit in der Wasserstoffperoxidlösung. Weiterhin ermöglicht das nach dem erfindungsgemäßen Verfahren hergestellte Granulat eine Anwendung und Lagerung ohne Staubentwicklung und weist eine konstante chemische Zusammensetzung auf, wodurch eine gleichbleibende und gegenüber dem Stand der Technik erheblich verbesserte Bleichwirkung ermöglicht wird.

Gegenstand der vorliegenden Erfindung ist daher weiterhin ein Mittel zum Entfärben oder Blondieren von Haaren, welches durch Vermischen einer wäßrigen Wasserstoffperoxidzubereitung mit einem nach dem vorstehend beschriebenen Verfahren hergestellten Granulat erhalten wird.

Das Granulat wird hierbei mit der Wasserstoffperoxidzubereitung in einem Gewichtsverhältnis von etwa 1 : 1 bis 1 : 3 vermischt, wobei ein Gewichtsverhältnis von 1 : 1,5 bis 1 : 2 besonders bevorzugt ist.

Die wäßrige Wasserstoffperoxidzubereitung enthält etwa 4 bis 18 Gewichtsprozent, vorzugsweise 6 bis 12 Gewichtsprozent, Wasserstoffperoxid sowie gegebenenfalls für derartige Zubereitungen übliche Zusatzstoffe, wie zum Beispiel Parfümöle, Farbstoffe, Stabilisatoren, Netzmittel und Emulgatoren.

Die Anwendung des so erhaltenen Mittels zum Entfärben oder Blondieren von Haaren erfolgt in üblicher Weise, indem man je nach Haarlänge 20 bis 40 Gramm, vorzugsweise 30 Gramm, des nach dem erfindungsgemäßen Verfahren hergestellten Granulates unmittelbar vor Gebrauch mit der Wasserstoffperoxidzubereitung in einem Gewichtsverhältnis von 1 : 1 bis 1 : 3, vorzugsweise 1 : 1,5 bis 1 : 2, vermischt, das so erhaltene Mittel zum Entfärben oder Blondieren von Haaren auf das Haar aufträgt und nach einer Einwirkzeit von 15 bis 60 Minuten wieder ausspült.

Die nachfolgenden Beispiele sollen den Gegenstand der Erfindung näher erläutern, ohne den Gegenstand auf diese Beispiele zu beschränken.

## Beispiele

### Beispiel 1: Herstellungsverfahren

In einem Pflugscharmischer, Typ FM 50 der Firma Gebrüder Lödige werden 15 kg des nachfolgenden pulverförmigen Gemisches bei Raumtemperatur durch Besprühen mit 2,25 kg (entsprechend 2,9 l) Isopropanol granuliert.

### Pulverfömiges Gemisch

| | |
|---|---|
| 400 g | Kaliumpersulfat |
| 100 g | Ammoniumpersulfat |
| 200 g | Natriumsilikat |
| 110 g | Magnesiumcarbonat |
| 80 g | Natriumstearat |
| 60 g | Polyvinylpyrrolidon |
| 37 g | Carboxymethylcellulose |
| 10 g | Natriumethylendiaminotetraacetat (EDTA) |
| 3 g | Parfüm |
| 1000 g | |

Nach Beendigung der Granulation (Granulationszeit : 10 Minuten) wird das erhaltene Granulat in einem Wirbelschichttrockner, Typ GPCG 3 der Firma Glatt getrocknet (Trocknungszeit : 25 Minuten). Die Zulufttemperatur beträgt hierbei etwa 60 Grad Celsius, wobei darauf geachtet wird, daß das Granulat nicht über 40 Grad Celsius erwärmt wird.

Das so erhaltene Granulat zeichnet sich durch eine hohe Abriebfestigkeit, eine gute Dispergierbarkeit, eine konstante chemische Zusammensetzung und eine ausgezeichnete Bleichwirkung aus.

### Beispiel 2: Herstellungsverfahren

15 kg des nachfolgenden pulverförmigen Gemisches

| | |
|---|---|
| 300 g | Kaliumpersulfat |
| 190 g | Ammoniumpersulfat |
| 220 g | Natriumsilikat |
| 110 g | Magnesiumcarbonat |
| 80 g | Natriumstearat |
| 60 g | Polyvinylpyrrolidon |
| 30 g | Carboxymethylcellulose |
| 10 g | Natriumethylendiaminotetraacetat (EDTA) |

$\overline{1000}\ \overline{g}$

werden in der in Beispiel 1 beschriebenen Weise durch Besprühen mit 2,1 kg (entsprechend 2,7 l) Isopropanol granuliert und getrocknet.

Es wird ein Granulat mit einer Korngrößenverteilung zwischen 0,15 und 1,2 mm erhalten.

**Beispiel 3: Vergleichsversuche**

Ein Blondiermittel, welches durch Vermischen von 10 g des Granulates gemäß Beispiel 1 beziehungsweise Beispiel 2 mit 15 g einer 9-prozentigen Wasserstoffperoxidlösungen hergestellt wurde, wurde bezüglich seiner Blondierwirkung mit einem Mittel gemäß der DE-AS 2 023 922, das durch Vermischen von 10 g des nachfolgend beschriebenen Granulates mit 15 g einer 9-prozentigen Wasserstoffperoxidlösung erhalten wurde, verglichen.

**Granulat gemaß der DE-AS 2 023 922**

In einem Pflugscharmischer, Typ FM 50 der Firma Gebrüder Lödige werden 14,1 kg des folgenden Pulvers

| | |
|---|---|
| 400 g | Kaliumpersulfat |
| 100 g | Ammoniumpersulfat |
| 200 g | Natriumsilikat |
| 110 g | Magnesiumcarbonat |
| 80 g | Natriumstearat |
| 37 g | Carboxymethylcellulose |
| 10 g | Natriummethylendiaminotetraacetat (EDTA) |
| 3 g | Parfüm |

$\overline{940}\ \overline{g}$

durch Besprühen mit einer Lösung von 0,9 kg Polyvinylpyrrolidon in 13,2 kg Isopropanol bei Raumtemperatur granuliert (Granulationszeit: 10 Minuten). Das Granulat wird sodann in der in Beispiel 1 beschriebenen Weise getrocknet.

Das so erhaltene Granulat ist äußerst hart und grobkörnig sowie in der Wasserstoffperoxidlösung nur schwer dispergierbar.

Die erhaltenen 3 Blondiermittel (Nr. 1: Granulat gemäß Beispiel 1; Hr. 2: Granulat gemäß Beispiel 2; Nr. 3: Granulat gemäß DE-AS 2 023 922) wurden auf braune Naturhaarsträhnen aufgetragen. Anschließend wurden die so behandelten Haarsträhnen 30 Minuten lang bei 40 Grad Celsius im Trockenschrank aufbewahrt und sodann mit Wasser gründlich ausgewaschen.

Die Farbmeßwerte der Haarsträhnen wurden mit Hilfe eines Farbmeßgerätes der Firma Minolta, Typ Chroma-Meter II ermittelt (siehe Tabelle 1).

Tabelle 1

| Lab-Farbmeßwerte von blondiertem Naturhaar | | | |
|---|---|---|---|
| | L | a | b |
| unbehandeltes Haar | 26,4 | 3,5 | 7,5 |
| mit Blondiermittel Nr. 1 behandeltes Haar | 67,7 | 4,3 | 34,0 |
| mit Blondiermittel Nr. 2 behandeltes Haar | 66,2 | 4,6 | 32,0 |
| mit Blondiermittel Nr. 3 behandeltes Haar | 43,4 | 5,2 | 22,5 |

Die mit dem erfindungsgemäßen Blondiermittel (Nr. 1 und Nr. 2) behandelten Haarsträhnen weisen sowohl gegenüber dem unbehandelten Haar als auch gegenüber dem mit dem aus der DE-AS 20 32 922 bekannten Blondiermittel (Nr.3) behandelten Haar einen erheblich höheren Helligkeitswert (L) und Gelbwert (b) auf, was eindeutig die bessere Blondierwirkung der erfindungsgemäßen Blondiermittel beweist.

Alle in der vorliegenden Anmeldung aufgeführten Prozentangaben stellen, soweit nicht anders vermerkt, Gewichtsprozente dar.

**Patentansprüche**

1. Verfahren zur Herstellung eines granulatförmigen Mittels zum Entfärben oder Blondieren von Haaren, dadurch gekennzeichnet, daß man in einer Granuliervorrichtung bei Raumtemperatur über einem mindestens ein anorganisches Persulfat und übliche Hilfs- und Zusatzstoffe sowie ein Bindemittel enthaltenden pulverförmigen Gemisch einen Alkohol versprüht und anschließend das erhaltene Granulat trocknet.

2. Verfahren nach Anspruch 1, dadurch gekennzeichnet, daß als Alkohol Ethanol, Isopropanol oder ein Gemisch dieser beiden Alkohole verwendet wird.

3. Verfahren nach Anspruch 1 oder 2, dadurch gekennzeichnet, daß das Verhältnis von pulverförmigem Gemisch zu Alkohol gleich 13 : 1 bis 4 : 1 ist.

4. Verfahren nach einem der Ansprüche 1 bis 3, dadurch gekennzeichnet, daß als Bindemittel Polyethylenglykole mit einem Molekulargewicht von 200 bis 10000 oder Polyvinylpyrrolidon verwendet werden.

5. Verfahren nach einem der Ansprüche 1 bis 4, dadurch gekennzeichnet, daß das Bindemittel in einer Menge von 1 bis 20 Gewichtsprozent in dem pulverförmigen Gemisch enthalten ist.

6. Verfahren nach einem der Ansprüche 1 bis 5, dadurch gekennzeichnet, daß die Granulationsdauer 10 bis 60 Minuten beträgt.

7. Verfahren nach einem der Ansprüche 1 bis 6, dadurch gekennzeichnet, daß das für die Granulation verwendete pulverförmige Gemisch 30 bis 65 Gewichtsprozent mindestens eines anorganischen Persulfates enthält.

8. Verfahren nach einem der Ansprüche 1 bis 7, dadurch gekennzeichnet, daß das anorganische Persulfat ausgewählt ist aus Natriumpersulfat, Kaliumpersulfat und Ammoniumpersulfat.

9. Granulatförmiges Mittel zum Entfärben oder Blondieren von Haaren, dadurch gekennzeichnet, daß es mit einem Verfahren nach einem der Ansprüche 1 bis 8 hergestellt wird.

**Claims**

1. Process for the production of an agent which is in granular form for removing the colour from or for bleaching hair, characterised in that in a granulator at room temperature an alcohol is sprayed over a mixture which is in the form of a powder and which contains at least one inorganic persulphate and conventional auxiliary and additional substances, in addition to a binding agent and, subsequently, drying the granulate obtained.

2. Process according to Claim 1, characterised in that ethanol, isopropanol or a mixture of these two alcohols is used as the alcohol.

3. Process according to Claim 1 or Claim 2, characterised in that the ratio of mixture in powder form to alcohol is 13:1 to 4:1.

4. Process according to any one of Claims 1 to 3, characterised in that polyethylene glycols with a molecular weight of from 200 to 10000, or polyvinylpyrolidone are used as the binding agent.

5. Process according to any one of Claims 1 to 4, characterised in that the binding agent is contained in an amount of from 1 to 20 weight % in the mixture which is in the form of a powder.

6. Process according to any one of Claims 1 to 5, characterised in that the duration of the granulation is from 10 to 60 minutes.

7. Process according to any one of Claims 1 to 6, characterised in that the mixture in the form of powder which is used for the granulation contains from 30 to 65 weight % of at least one inorganic persulphate.

8. Process according to any one of Claims 1 to 7, characterised in that the inorganic persulphate is selected from sodium persulphate, potassium persulphate and ammonium persulphate.

9. Composition which is in granular form for removing the color from hair or for bleaching hair, characterized in that it is produced by a process according to any one of claims 1 to 8.

**Revendications**

1. Procédé de préparation d'un agent granuleux destiné à la coloration ou au blondissement des cheveux, caractérisé en ce qu'on pulvérise dans un dispositif de granulation à la température ambiante un alcool sur un mélange pulvérulent contenant au moins un persulfate minéral et des additifs ou adjuvants usuels ainsi qu'un liant, puis on sèche le granulat obtenu.

2. Procédé selon la revendication 1, caractérisé en ce qu'on choisit comme alcool, l'éthanol, l'isopropanol ou un mélange de ces deux alcools.

3. Procédé selon la revendication 1 ou 2, caractérisé en ce que la teneur en mélange pulvérulent en alcool va de 13 : 1 à 4 : 1.

4. Procédé selon une des revendications 1 à 3, caractérisé en ce qu'on utilise comme liant le polyéthylèneglycol avec une masse moléculaire de 200 à 10000, ou de la polyvinylpyrrolidone.

5. Procédé selon l'une des revendications 1 à 4, caractérisé en ce que le liant est contenu à raison de 1 à 20% en poids du mélange pulvérulent.

6. Procédé selon l'une des revendications 1 à 5, caractérisé en ce que le temps de granulation va de 10 à 60 minutes.

7. Procédé selon l'une des revendications 1 à 6, caractérisé en ce que le mélange pulvérulent utilisé pour la granulation, contient de 30 à 65% en poids d'au moins un persulfate minéral.

8. Procédé selon l'une des revendications 1 à 7, caractérisé en ce que le persulfate minéral est choisi parmi le persulfate de sodium, le persulfate de potassium et le persulfate d'ammonium.

9. Agent granuleux de coloration ou de blondissement des cheveux, caracterisé en ce qu'il est obtenu par le procédé selon l'une des revendications 1 à 8.